# EUROPEAN PATENT SPECIFICATION

(11) **EP 1 494 656 B1**
(45) Date of publication and mention of the grant of the patent: **18.06.2008**
(21) Application number: 03715908.4
(22) Date of filing: 10.04.2003
(51) Int. Cl.: A61K 9/22, A61K 31/485

(54) **ANALGESIC ORAL COMPOSITION WITH CONTROLLED RELEASE OF AN OPIOID**
ANALGETISCHE ORALE ZUSAMMENSETZUNG MIT GESTEUERTER FREISETZUNG EINES OPIOIDS
COMPOSITION ORALE ANALGESIQUE AVEC LIBERATION CONTROLEE D'UN OPIOIDE

(30) Priority: 12.04.2002 SK 5012002
(43) Date of publication of application: 12.01.2005
(73) Proprietor: Zentiva, A.S, 920 27 Hlohovec (SK)
(72) Inventor: RAZUS, Luboslav, 920 01 Hlohovec (SK); GATTNAR, Ondrej, 841 02 Bratislava (SK); VARGA, Ivan, 920 01 Hlohovec (SK); LEHOCKY, Mikulas, 920 01 Hlohovec (SK); KORMANOVA, Viera, 920 01 Hlohovec (SK); HUBINOVA, Viera, 920 01 Hlohovec (SK)
(74) Representative: Jirotkova, Ivana
(86) International application number: PCT/SK2003/000008
(87) International publication number: WO 2003/086365

(56) References cited:
- EP-A- 0 249 347
- WO-A-01/32148
- WO-A-01/47497
- WO-A-98/14176
- DE-A- 19 747 261
- EL-SAYED G M ET AL: "KINETICS OF THEOPHYLLINE RELEASE FROM DIFFERENT TABLET MATRICES" STP PHARMA SCIENCES, PARIS, FR, vol. 6, no. 6, 1 November 1996 (1996-11-01), pages 390-397, XP000676942 ISSN: 1157-1489

## Description

### Technical Field

The invention falls under the area of pharmacy and it addresses a composition and a method for preparing a therapeutic formulation containing, as the active substance, dihydrocodeine and/or its pharmaceutically acceptable salts in the form of tablets with controlled release of this active substance that belongs to the therapeutic group of strongly potent analgesics.

### State of the Art

A composition and a method of manufacturing an oral composition with controlled release of dihydrocodeine tartrate is illustrated in EP 0 249 347 owned by Euroceltique SA. The product is a matrix tablet containing, in addition to the active substance, also suitable matrix-forming materials that provide for gradual release of dihydrocodeine tartrate, mass equivalents of dihydrocodeine base or other dihydrocodeine salts.

Preferably used are hydroxyethyl cellulose, cetostearyl alcohol, anhydrous lactose, talc and magnesium stearate.

The manufacturing principle is the granulation of the active substance in admixture with lactose and hydroxyethyl cellulose and water, drying out of the granulate, followed by adding of melted cetostearyl alcohol at an elevated temperature. After mixing up, followed by cooling the mixture down with air, re-granulating and sieving the granulate, talc and magnesium stearate is added. Tablets are compressed out from this mixture.

An disadvantage of the above specification is the use of two materials forming the matrix for gradual release of the active substance. These are hydroxyethyl cellulose and cetostearyl alcohol. The key disadvantage is the need to perform the basic technological operation when hot, which requires a granulation device having the possibility of heating of the processed material and a need of use of melted cetostearyl alcohol which, in the course of intensive stirring, forms a coat on the particles of the granulate of the active substance, lactose and hydroxylethyl cellulose. After the mixture is cooled down, properly treated and compressed into tablets, the required course of release of the active substance from the therapeutic formulation prepared in the above-described manner is achieved. WO 01/32148 discloses a controlled release formulation of hydrocodone, containing a mixture of adjuvants including stearyl alcohol and microcrystalline cellulose. A controlled release formulation of tramadol is disclosed in WO 01/47497, wherein the adjuvants also include stearyl alcohol and microcrystalline cellulose.

### Substance of the Invention

The above described disadvantages are addressed in this invention, according to which oral tablets with controlled release containing a therapeutic dose of dihydrocodeine and/or an equivalent amount of other therapeutically acceptable dihydrocodeine salts are prepared using a new, simpler and less demanding technological process that does not require any special manufacturing device for the granulation with a supply of heat energy and without any need of incorporating auxiliary materials influencing release of the therapeutic agent in the molten state.

The required profile of release of the active substance after compressing into tablets is achieved by using a single adjuvant responsible for the required effect and a pharmaceutical water-insoluble filler and by disintegrating a portion of the pressings through a sieve having a proper mesh size and mixing the disintegrate with the primary mixture in an experimentally verified ratio.

The oral tablets prepared using the method of the invention comply with the requirements for the release and they do not need any additional treatment such as coating with a film. They can be divided by a line to facilitate possible breaking into smaller doses without any impact of the tablet breaking upon the course of released of the active substance used.

Another advantage of the below described invention is the possibility of processing of the mixture into pressings having any required therapeutic content of the active substance and any shape, the release profile being maintained.

The principle of the manufacture is simple mixing of the active substance with adjuvants, moistening of the mixture during stirring until formation of the required structure of the granulate, its drying and proper sizing of the particles of the granulate, addition of a lubricant facilitating the compressing process and forming of tablets of required weight, shape and fracture resistance that are chemically and physically stable for a sufficient period of time and can be inserted into commonly used pharmaceutical packages without problems.

Accordingly, the invention consists in an analgesically active oral therapeutic composition with controlled release, characterized in that it contains dihydrocodeine and/or its pharmaceutically or therapeutically acceptable salt as the active substance, micronized ester of glycerol and behenic acid ensuring said controlled release, calcium phosphate dihydrate and further adjuvants, the composition being prepared by blending the active substance in admixture with the micronized glyceryl behenate with calcium phosphate dihydrate, moistening the mixture with a solution of a co-polymer of vinylpyrrolidone and vinylacetate in the ratio 6 : 4 having the relative molecular weight from 45,000 to 70,000 in a mixture of ethyl alcohol and water, compressing 1 to 99 % by weight of the resulting agglomerate into pressings of any shape and weight having the fracture resistance from 40 N to 110 N, disintegrating said pressings by the oscillation method through a sieve having the size of openings from 0.65 to 0.8 mm and thoroughly homogenizing the disintegrate with previously not compressed and not disintegrated residue of the prepared lot.

The analgesically active oral therapeutic composition with controlled release and the method for its preparation according to this invention comprise, in addition to dihydrocodeine tatrate and/ or dihydrocodeine base and/or pharmaceutically salts of dihydrocodeine, other adjuvants suitable for pharmaceutical formulations, namely:
a) Micronized glyceryl ester of behenic acid, having the particle size ranging from 1 to 100 µm, preferably having such particle size distribution that 90 % of them is sized from 1.5 to 60 µm.
   It has been found out experimentally and verified that the most beneficial content of the glyceryl ester of behenic acid, to achieve the required properties of the therapeutic composition according to this invention is from 10 to 65 % by weight, preferably from 20 to 50 % by weight of the tablet.
b) Calcium phosphate dihydrate, in an amount from 10 to 60 % by weight, preferably from 15 % to 50 % by weight of the tablet.
c) A copolymer of vinylpyrrolidone and vinylacetate in the ratio of 6 : 4, having the relative molecular weight from 45,000 to 70,000, in an amount from 1.0 to 6 % by weight, preferably from 1.5 % to 4,0 % by weight
d) A micronized alkali salt of fumaric acid and stearyl alcohol, preferably the sodium salt of the ester of fumaric acid and stearyl alcohol, having the particle size up to 10 µm, preferably up to 8 µm, in an amount from 0.2 to 3 % by weight, preferably from 0.8 to 1,5 % by weight.

It has been surprisingly found out and confirmed that the profile of the release of the active substance from the therapeutic composition according to the described invention can be set as required by modifying the ratio of the glyceryl ester of behenic acid and calcium phosphate dihydrate in the tablet.

It has been further found out and confirmed that by disintegrating the compacts through a properly sized sieve and the re-compressing of the ground material a surprisingly rapid drop in the rate of the release of the active substance is obtained. Combining amounts of the primary mixture for the compression of tablets and of the disintegrated mixture permits to influence the process of release of the active substance from the tablet.

The essence of the manufacture of the solid oral dosage form of this invention resides in mixing, in a high-speed pharmaceutical homogenizer for wet homogenisation, the active substance together with micronized glyceryl ester of behenic acid having the particle size from 1.5 to 60 µm, in an amount from 20 to 50 % by weight and with alkali salts of phosphoric acid, preferably using calcium phosphate dihydrate, in an amount from 15 to 50 % by weight. This mixture is gradually moistened with a solution of a vinylpyrrolidone-vinylacetate co-polymer in the ratio 6 : 4 having the relative molecular mass weight from 45,000 to 70,000 in an amount from 1.5 to 4.0 % by weight in a mixture of ethyl alcohol and water wherein ethyl alcohol accounts for 10 to 80 % by volume, preferably from 25 to 70 % by volume. The mixture is shortly stirred until agglomerate is formed.

Thus prepared agglomerate is box dried, or fluidization dried, or vacuum dried, or microwave dried, preferably fluidization dried, such that the final humidity is in the range from 0.1 to 2.0 % by weight, preferably from 0.5 to 1.5 % by weight. The temperature of the product when dried has to reach 35 to 42 °C, preferably 39 to 41 °C.

The dried agglomerate is subjected to resizing of the particles to match the requirements of the tablet compressing process and is homogenized with the micronized sodium salt of the ester of stearyl alcohol with fumaric acid. After thorough homogenisation, the mixture is ready for the tablet compressing process.

In order to achieve the required profile of release of the active substance from the tablet, 1 to 99 % by weight, preferably 65 to 85 % by weight of the finished mixture for compressing is compressed into pressings of any shape and weight having the fracture resistance from 40 N to 110 N, preferably from 60 N to 85 N, said pressings are disintegrated by the oscillation method through a sieve having the size of openings from 0.65 to 0.8 mm, preferably 0.7 mm, and the disintegrate is thoroughly homogenized with previously not compressed and not disintegrated residue of the prepared lot.

The mixture is compressed in rotary tablet forming machines into tablets having the required contents of the active substance, the fracture resistance of the tablets being from 40 to 110 N, preferably from 60 to 85 N. The tablets may be flat round-shaped, lens round-shaped, of an oval or other shape, with possible presence of a break line on one or two surfaces of the tablet.

Thus obtained tablets can be adjusted into versatile packages destined for pharmaceutical products, preferably into a blister package consisting of a PVC/PVDC combination of heat formed sheet and an aluminium foil coated with an adhesive material.

### Examples

The subject-matter of the invention is illustrated in, but not limited by, the following examples.

### Example 1

a) Tablet composition

| | |
|---|---|
| Dihydrocodeine tartrate | 33.33 % |
| Behenic acid glyceryl ester | 46.66 % |
| Calcium phosphate dihydrate | 16.39 % |
| Vinylpyrrolidone-vinylacetate co-polymer | 2.51 % |
| Sodium stearyl fumarate | 1.11 % |

b) Process of preparing
Dihydrocodeine tartrate is, in a suitable type of a pharmaceutical granulator, blended with micronized glyceryl ester of behenic acid having the particle size from 1 to 60 µm and with calcium phosphate dihydrate. The blended mixture is moistened gradually with a solution of a vinylpyrrolidone-vinylacetate co-polymer in the ratio 6:4 in a mixture of 48 % ethyl alcohol - water until agglomerate forms. The agglomerate is transferred to the vessel of a device for fluidization drying and is dried under moderate fluidization at the temperature of the supplied air 55 °C until the temperature of the product reaches 40 °C, which at the same time indicates completion of the drying process.
The dried out granulate is subjected to resizing by means of oscillation sieving with a sieve having openings from 0.65 to 0.8 mm and it is homogenized in a dry homogenizer device with a lubricant, namely micronized sodium stearyl fumarate.
At this point, the mixture is ready for compressing in rotary tablet forming machines into tablets having the required contents of dihydrocodeine tartrate and the required fracture resistance.
c) Dissolution test results (USP Paddle Method 900 ml, 100 rpm, aq. buffer pH 1.6-7.2)

| 1 hour | 2 hours | 3 hours | 4 hours | 5 hours | 6 hours | 7 hours | 8 hours |
|---|---|---|---|---|---|---|---|
| 36.90 % | 64.60 % | | 100 % | | | | |

### Example 2

a) Tablet composition

| | |
|---|---|
| Dihydrocodeine tartrate | 33.33 % |
| Behenic acid glyceryl ester | 16.39 % |
| Calcium phosphate dihydrate | 46.66 % |
| Vinylpyrrolidone-vinylacetate co-polymer | 2.51 % |
| Sodium stearyl fumarate | 1.11 % |

b) Process of preparing: same as in ***Example 1***
c) Results of the dissolution test

| 1 hour | 2 hours | 3 hours | 4 hours | 5 hours | 6 hours | 7 hours | 8 hours |
|---|---|---|---|---|---|---|---|
| | | | | | | | |
| 47.1 % | 62.8 % | | 83.7 % | | 92.4 % | | 93.6% |

### Example 3

*a)* Tablet composition:

| | |
|---|---|
| Dihydrocodeine tartrate | 30.15 % |
| Behenic acid glyceryl ester | 28.90 % |
| Calcium phosphate dihydrate | 37.69 % |
| Vinylpyrrolidone-vinylacetate co-polymer | 2.26 % |
| Sodium stearyl fumarate | 1.00 % |

*b)* Process of preparing: same as in ***Example 1***
*c)* Results of the dissolution test:

| 1 hour | 2 hours | 3 hours | 4 hours | 5 hours | 6 hours | 7 hours | 8 hours |
|---|---|---|---|---|---|---|---|
| 37.99 % | 53.63 % | 64.53 % | 72.92 % | 79.50 % | 84.86 % | 89.09 % | 92.54 % |

### Example 4

a) Tablet composition:
Same as in ***Example 1***
b) Process of preparing: same as in ***Example 1***
until the step of compressing the tablets from the finished granulate, of which a 25 % by weight amount is left, the remaining 75 % are compressed into pressings, which are disintegrated by the oscillation method through a sieve having the size of openings from 0.65 mm to 0.80 mm. After the disintegration, both products are homogenized in a homogenizer for dry pharmaceutical homogenising and the resulting product is subjected to the compression process, thus giving tablets that have the required contents of the active substance and the required shape and fracture resistance.
b) Results of the dissolution test:

| 1 hour | 2 hours | 3 hours | 4 hours | 5 hours | 6 hours | 7 hours | 8 hours |
|---|---|---|---|---|---|---|---|
| 32.0% | 45.9% | | 65.2% | | 79.5% | | 94.8% |

### Industrial Applicability

The invention may be used in the pharmaceutical industry in the manufacture of therapeutic composition containing gradually released analgesically active therapeutic agent dihydrocodeine and/or its pharmaceutically acceptable salts.

The composition according to this invention is useful in alleviating moderately strong to strong pains, such as post-surgery pains, post-injury pains, chronic pains in the spine, severe arthritis, acute osteoporosis, neurogenic pains, pains related to cancer and other pains.

## Claims

1. An analgesically active oral therapeutic composition with controlled release, **characterized in that** it contains dihydrocodeine and/or its pharmaceutically or therapeutically acceptable salt as the active substance, micronized ester of glycerol and behenic acid ensuring said controlled release, calcium phosphate dihydrate and further adjuvants, the composition being prepared by blending the active substance in admixture with the micronized glyceryl behenate with the alkali salt of phosphoric acid, moistening the mixture with a solution of a co-polymer of vinylpyrrolidone and vinylacetate in the ratio 6 : 4 having the relative molecular weight from 45,000 to 70,000 in a mixture of ethyl alcohol and water, compressing 1 to 99 % by weight of the resulting agglomerate into pressings of any shape and weight having the fracture resistance from 40 N to 110 N, disintegrating said pressings by the oscillation method through a sieve having the size of openings from 0.65 to 0.8 mm and thoroughly homogenizing the disintegrate with previously not compressed and not disintegrated residue of the prepared lot.

2. The composition according to claim 1 **characterized in that** the contents of the active substance therein is 25 to 40 % by weight, preferably 27 to 35 % by weight.

3. The composition according to any of preceding claims **characterized in that** the adjuvants are calcium phosphate dihydrate in an amount from 10 to 60 % by weight, preferably from 15 to 50 % by weight, the glyceryl ester of behenic acid having the particle size from 1 to 100 µm, preferably such that 90 % of the particles is of a size from 1.5 to 60 µm, and being in an amount from 10 to 60 % by weight, preferably from 20 to 50 % by weight, a copolymer of vinylpyrrolidone and vinylacetate in the ratio 6 : 4 having the relative molecular weight from 45,000 to 70,000 in an amount from 1 to 6 % by weight, preferably from 1.5 to 4 % by weight, and a micronized sodium salt of the ester of fumaric acid with stearyl alcohol having the particle size up to 10 µm, preferably up to 8 µm, in an amount from 0.2 to 3 % by weight, preferably from 0.8 to 1.5 % by weight.

4. A method for preparing a therapeutic composition according to claim 1 **characterized in that** the active substance is blended in admixture with a micronized ester of glycerol and behenic acid with calcium phosphate dihydrate, during blending the mixture is moistened with a solution of a co-polymer of vinylpyrrolidone and vinylacetate in the ratio 6 : 4 having the relative molecular weight from 45,000 to 70 ,000 in a mixture of ethyl alcohol and water, thus the mixture being agglomerated, and thereafter, 1 to 99 % by weight, preferably 65 to 85 % by weight of the agglomerate is compressed into pressings of any shape and weight having the fracture resistance from 40 N to 110 N, preferably from 60 N to 85 N, said pressing are disintegrated by the oscillation method through a sieve having the size of openings from 0.65 to 0.8 mm, preferably 0.7 mm, and the disintegrate is thoroughly homogenized with previously not compressed and not disintegrated residue of the prepared lot.

## Patentansprüche

1. Analgetisch wirkende orale Arzneizusammensetzung mit kontrollierter Freisetzung, **dadurch gekennzeichnet, dass** sie Dihydrokodein und/oder sien pharmazeutisch oder therapeutisch akzeptables Salz als Wirkstoff, den mikronisierte Ester von Glycerol und Behensäure, versichernd die kontrollierte Freisetzung, Calciumphosphatdihydrat und weitere Zusätze enthält, wobei die Zusammensetzung durch Mischung des Wirkstoffes im Gemisch mit mikronisierten Glycerylbehenat mit dem Alkalisalz der Phosphorsäure, Befeuchten der Mischung mit der Lösung des Co-polymers von Vinylpyrrolidon und Vinylacetat im Verhältnis 6 : 4 mit relativem Molekulargewicht von 45.000 bis 70,000 in der Mischung von Ethylalkohol und Wasser, Komprimieren 1 bis 99 Gew.-% des entstehenden Agglomerats in Pressungen von jeder Form und Größe, die eine Bruchfestigkeit von 40 N bis 110 N haben, Abbauen der genanten Pressungen durch die Schwingungsmethode mit einem Sieb mit Öffnungen von 0.65 bis 0.8 mm und gut Homogenisierung des Abbausprodukt mit den vorher nicht komprimierten und nicht abgebauten Rückständen des hergestellten Anteils hergestellt wird.

2. Zusammensetzung nach Anspruch 1, **dadurch gekennzeichnet, dass** der Gehalt des Wirkstoffes darin 25 bis 40 Gew.-%, vorzugsweise 27 bis 35 Gew.-%, beträgt.

3. Zusammensetzung nach einer der vorhergehenden Ansprüche, **dadurch gekennzeichnet, dass**, dass die Zusätze Calciumphosphatdihydrat in der Menge von 10 bis 60 Gew.-%, vorzugsweise von 15 bis 50 Gew.-%, der Glycerylester der Behensäure mit Teilchengröße von 1 bis 100 µm, vorzugsweise so dass 90 % der Teilchen die Größe von 1.5 bis 60 µm haben und in der Menge von 10 bis 60 Gew.-%, vorzugsweise von 20 bis 50 Gew.-% sind, das Copolymer von Vinylpyrrolidon und Vinylacetat im Verhältnis von 6 : 4, mit relativem molekularem Gewicht von 45,000 bis 70,000 in der Menge von 1 bis 6 Gew.-%, vorzugsweise von 1.5 bis 4Gew.-%, und das mikronisierte Natriumsalz des Esters der Fumarsäure mit Stearylalkohol mit der Teilchengröße von bis zu 10 µm, vorzugsweise bis zu 8 µm, in der Menge von 0.2 bis 3 Gew.-%, vorzugsweise von 0.8 bis 1.5 Gew.-%, sind.

4. Verfahren zur Herstellung der Arzneimittelzusammensetzung nach Anspruch 1, **dadurch gekennzeichnet, dass** der Wirkstoff unter Beimischung mit dem mikronisierten Ester von Glycerol und Behensäure mit Calciumphosphatdihydrat gemischt wird, während der Mischung das Gemisch mit der Lösung vom Copolymer aus Vinylpyrrolidon und Vinylacetat im Verhältnis von 6 : 4 mit relativem Molekulargewicht von 45.000 bis 70.000 in einer Mischung von Ethylalkohol und Wasser befeuchtet wird, somit die Mischung agglomeriert wird, und daraufhin 1 bis 99 Gew.-%, vorzugsweise 65 bis 85 Gew.-% des Agglomerats in Pressungen von jeder Form und Größe mit Bruchfestigkeit von 40 N bis 110 N, vorzugsweise von 60 N bis 85 N, komprimiert werden, die genannten Pressungen durch die Schwingungsmethode mit einem Sieb mit Öffnungen von 0.65 bis 0.8 mm, vorzugsweise 0,7 mm, abgebaut werden und das Abbauprodukt mit den vorher nicht komprimierten und nicht abgebauten Rückständen des hergestellten Anteils gut homogenisiert wird.

## Revendications

1. Composition thérapeutique orale active analgésiquement, à libération contrôlée, **caractérisée en ce qu'**elle contient la dihydrocodeïne et / ou son sel pharmaceutiquement ou thérapeutiquement acceptable comme substance active, un ester de glycérol et d'acide béhénique micronisé assurant ladite libération contrôlée, le dihydrate de phosphate de calcium et autres additifs, la composition étant préparée en mélangeant la substance active en mélange avec le béhénate de glycérol micronisé avec le sel alcalin d'acide phosphorique, humidifiant le mélange avec une solution de co-polymère de vinylpyrrolidone et de vinylacétate dans la proportion de 6 : 4 ayant le poids moléculaire rélatif de 45,000 à 70,000 dans un mélange d'alcool éthylique et d'eau, comprimant 1 à 99 % en poids de l'agglomérat résultant en moulages de n'importe quelle forme ayant une résistance de fracture comprise entre 40 N et 110 N, désintégrant lesdits moulages par la méthode d'oscillation dans un tamis de 0.65 à 0.8 mm d'ouverture puis homogénéisant soigneusement le désintégrat avec le résidu du lot préparé non compressé ni désintédré préalablement.

2. La composition selon la revendication 1, **caractérisée en ce que** le contenu de la substance active est de 25 à 40 % en poids, de préférence 27 à 35 % en poids.

3. La composition selon l'une quelconque des revendications précédentes, **caractérisée en ce que** les additifs sont le dihydrate de phosphate calcium dans une proportion de 10 à 60 % en poids, de préférence 15 à 50 % en poids, l'ester glycérylique d'acide béhénique ayant la grosseur de particules de 1 à 100 µm, de préférence telle que 90 % des particules ont la grosseur comprise entre 1.5 et 60 µm, et présent dans une proportion de 10 à 60 % en poids, de préférence 20 à 50 % en poids, un co-polymère de vinylpyrrolidone et de vinylacétate dans un ration de 6 : 4 d'un poids moléculaire rélatif compris entre 45,000 et 70,000 dans une proportion de 1 à 6 % en poids, de préférence 1.5 à 4 % en poids, et un sel de sodium micronisé d'ester d'acide fumarique et alcool stéaryle avec une grosseur de particules jusqu'à 10 µm, de préférence jusqu'à 8 µm, dans une proportion comprise entre 0.2 et 3 % en poids, de préférence 0.8 à 1.5 % en poids.

4. Procédé de préparation de la composition thérapeutique selon la revendication 1, **caractérisé en ce que** la substance active est mélangée en mélange avec l'ester de glycérol et acide béhénique micronisé avec le dihydrate de calcium phosphate, pendant le mélange, le mélange est humidifiée avec une solution de co-polymère de vinylpyrrolidone et de vinylacétate dans la proportion de 6 : 4 ayant le poids moléculaire rélatif de 45,000 à 70,000 dans un mélange d'alcool éthylique et d'eau, de façon que le mélange est agglomeré, puis de 1 à 99 % en poids, de préférence 65 à 85 % en poids de l'agglomérat est comprimé en moulages de n'importe quelle forme et poids ayant une résistance de fracture comprise entre 40 N et 110 N, de préférence entre 60 N et 85 N, lesdits moulages sont désintegrés par la méthode d'oscillation à travers un tamis de 0.65 à 0.8 mm, de préférence 0.7 mm, et le désintégrat est homogénéisé soigneusement avec le résidu du lot préparé non compressé ni désintédré préalablement.
